# EUROPEAN PATENT APPLICATION

(11) **EP 4 491 153 A1**
(43) Date of publication of application: **15.01.2025**
(21) Application number: 23765759.8
(22) Date of filing: 17.02.2023
(51) Int. Cl.: A61F 2/24

(54) **TRICUSPID VALVE REPAIR DEVICE**

(30) Priority: 10.03.2022 CN 202210228343
(71) Applicant: Shanghai Shenqi Medical Technology Co., Ltd., Shanghai 201203 (CN)
(72) Inventor: SONG, Rui, Shanghai 201203 (CN); FAN, Jun, Shanghai 201203 (CN); WANG, Sen, Shanghai 201203 (CN); ZHOU, Guolei, Shanghai 201203 (CN); DAI, Zhihao, Shanghai 201203 (CN)
(74) Representative: Cabinet Laurent & Charras
(86) International application number: PCT/CN2023/076774
(87) International publication number: WO 2023/169181

(57) **Abstract**

A tricuspid valve repair device, comprising a guiding catheter (1), a first adjusting mechanism (2), a delivery catheter (3), a second adjusting mechanism (4), a pushing rod (5) and a repair clip (7); the first adjusting mechanism (2) is used for adjusting a bending angle of a distal end of the guiding catheter (1) in a first direction and a second direction which are perpendicular to each other; the delivery catheter (3) is movably arranged in the guiding catheter (1); the second adjusting mechanism (4) is used for adjusting a bending angle of the delivery catheter (3) relative to the guiding catheter (1); the pushing rod (5) is movably arranged in the delivery catheter (3); the repair clip (7) is detachably arranged at a distal end of the pushing rod (5). A movable space range of the repair clip (7) is enlarged and a repair treatment range of the repair clip (7) on the tricuspid valve is therefore improved, so that the operation difficulty of a delivery system is reduced to a great extent, and the dependence of a tricuspid valve surgery on the technical level and clinical experience of a doctor is therefore reduced.

## Description

### CROSS-REFERENCE TO RELATED APPLICATIONS

The present application claims priority to Chinese patent application No. 2022102283438, entitled "Tricuspid Valve Repair Device", filed on March 10, 2022, the disclosure of which is hereby incorporated by reference in its entirety.

### TECHNICAL FIELD

The present application relates to a field of medical devices and, in particular, to a tricuspid valve repair device.

### BACKGROUND

With socio-economic development and population ageing, the incidence rate of valvular heart disease increased significantly. A tricuspid valve is located in an opening between a right atrium and a right ventricle. The tricuspid valve opens to allow blood from the right atrium to fill the right ventricle, and when the right ventricle contracts to pump the blood into lungs, the tricuspid valve closes to prevent blood from flowing back into the right atrium. When the tricuspid valve does not completely close, some blood leaks back into the right atrium through the tricuspid valve with each contraction of the right ventricle, which is referred to as tricuspid regurgitation. Conventional valvular interventional therapy techniques are, based on an edge-to-edge surgical principle, employed to deliver a valve clamp to a target tricuspid valve, and clamp the leaflets of the tricuspid valve by means of the relative opening and closing of clamp plates, so as to relatively fix the leaflets of the tricuspid valve, and thus reduce a gap between the leaflets, and reduce tricuspid regurgitation.

At present, conventional surgical treatment is still the preferred treatment for patients with severe valvular disease. However, for patients with advanced age, multiple organ disease, history of thoracotomy, and poor cardiac function, conventional surgical treatment is high in risk and mortality rate, and some patients do not even have the chance of surgery. Transcatheter valve replacement/repair has attracted widespread attention from experts and scholars due to its advantages of no thoracotomy, less trauma, and rapid patient recovery. Due to the complexity of human body structure, the accuracy of interventional surgical positioning is particularly important. In order to accurately deliver an implant to the target site through blood vessels with different pathways, a controlled bend delivery device is usually used.

The conventional controlled bend technique is implemented by embedding a controlled bend wire on a side of a controlled bend catheter. A distal end of the controlled bend wire is welded to a pull ring at a distal end of the controlled bend catheter. A proximal end of the controlled bend wire is connected to a handle. For example, the controlled bend wire is fixed on a slider. The slider can be controlled to move linearly to pull the controlled bend wire, so as to control the controlled bend wire to bent. The conventional controlled bend technique cannot control a controlled bend rate. Since the controlled bend wire is driven by the slider, the size of the handle in its length direction is large. The overall structure of the handle is not compact enough, the movable range thereof is small, the operation difficulty is large, and it is overly dependent on the skill level and clinical experience of the doctor.

### SUMMARY

Accordingly, the present application provides a tricuspid valve repair device, which reduces the operation difficulty of the delivery system, and reduces the dependence of the tricuspid valve repair procedure on the doctor's skill level and clinical experience.

To achieve the above objects, the following technical solutions are adopted in this application.

A tricuspid valve repair device, including:
a guide catheter;
a first adjusting mechanism configured to adjust bending angles of a distal end of the guide catheter in a first direction and a second direction;
a delivery catheter movably disposed in the guide catheter;
a second adjusting mechanism configured to adjust a bending angle of the delivery catheter with respect to the guide catheter;
a push rod movably disposed in the delivery catheter; and
a repair clip detachably disposed at a distal end of the push rod.

In an embodiment, the first adjusting mechanism includes a first operating handle, a first adjusting member and a first pulling wire. A proximal end of the guide catheter is arranged on the first operating handle. An end of the first pulling wire is connected to the first adjusting member. Another end of the first pulling wire is connected to the distal end of the guide catheter. The first adjusting member is arranged on the first operating handle. The first adjusting member is configured to pull or release the first pulling wire to adjust the bending angle of the distal end of the guide catheter in the first direction.

In an embodiment, the first adjusting mechanism further includes a first fixing member disposed at the distal end of the guide catheter. The other end of the first pulling wire is connected to the first fixing member.

In an embodiment, two first pulling wires are symmetrically arranged with respect to an axis of the guide catheter, and are respectively connected to two ends of the first fixing member in a radial direction.

In an embodiment, the first fixing member is a fixing ring. A first limiting protrusion is formed on one of an inner wall of the distal end of the guide catheter and the first fixing member, and a first limiting groove, which matches with the first limiting protrusion, is formed on another one of the inner wall of the distal end of the guide catheter and the first fixing member.

In an embodiment, a first marker band abutting the first fixing member is disposed at the distal end of the guide catheter. The first marker band is located on a side of the first fixing member away from the distal end of the guide catheter.

In an embodiment, the first adjusting member includes a first base body and a first rotating shaft. The first base body is disposed on the first operating handle. The first base body is of a hollow structure. An end of the first rotating shaft is rotatably arranged in the first base body. Another end of the first rotating shaft extends out of the first base body, and has a first steering part provided thereon. The first pulling wire extends into the first base body and is wrapped on the first rotating shaft.

In an embodiment, the first steering part is correspondingly provided with a scale.

In an embodiment, the first adjusting mechanism further includes a second adjusting member and a second pulling wire. An end of the second pulling wire is connected to the second adjusting member. Another end of the second pulling wire is connected to the distal end of the guide catheter. The second adjusting member is arranged on the first operating handle. The second adjusting member is configured to pull or release the second pulling wire to adjust the bending angle of the distal end of the guide catheter in the second direction.

In an embodiment, a plurality of pulling wire cavities extending in an axial direction of the guide catheter are formed on a tube wall of the guide catheter. The first pulling wire and the second pulling wire are slidably inserted through the plurality of pulling wire cavities, respectively.

In an embodiment, the second adjusting member includes a second base body and a second rotating shaft. The second base body is disposed on the second operating handle. The second base body is of a hollow structure. An end of the second rotating shaft is rotatably arranged in the second base body. Another end of the second rotating shaft extends out of the second base body, and has a second steering part provided thereon. The second pulling wire extends into the second base body and is wrapped on the second rotating shaft.

In an embodiment, the first adjusting mechanism further includes a first fixing member disposed at the distal end of the guide catheter. Two second pulling wires are symmetrically arranged with respect to an axis of the guide catheter, and are respectively connected with two ends of the first fixing member in a radial direction.

In an embodiment, a hardness of a proximal end of the guide catheter is greater than a hardness of the distal end of the guide catheter.

In an embodiment, at a bend portion in the first direction, a hardness of an inner side of the bend portion of the guide catheter is greater than a hardness of an outer side of the bend portion of the guide catheter.

In an embodiment, at a bend portion in the first direction, a curvature of the guide catheter is greater than a curvature of the delivery catheter.

In an embodiment, the first adjusting mechanism is further configured to adjust bending angles of the distal end of the guide catheter in a third direction and a fourth direction. The first direction and the third direction are in a first plane, the second direction and the fourth direction are in a second plane, and the first plane is perpendicular to the second plane.

In an embodiment, the first adjusting mechanism includes four pulling wires configured for controlling the bending angles of the distal end of the guide catheter in the first direction, the second direction, the third direction, and the fourth direction, respectively.

In an embodiment, the second adjusting mechanism includes a second operating handle, a third adjusting member and a third pulling wire. A proximal end of the delivery catheter is arranged on the second operating handle. An end of the third pulling wire is connected to the third adjusting member. Another end of the third pulling wire is connected to the distal end of the delivery catheter. The third adjusting member is arranged on the second operating handle. The third adjusting member is configured to pull or release the third pulling wire to adjust a bending angle of the delivery catheter with respect to the guide catheter.

In an embodiment, the third adjusting member includes a third base body and a third rotating shaft. The third base body is disposed on the third operating handle. The third base body is of a hollow structure. An end of the third rotating shaft is rotatably arranged in the third base body. Another end of the third rotating shaft extends out of the third base body, and has a third steering part provided thereon. The third pulling wire extends into the third base body and is wrapped on the third rotating shaft.

In an embodiment, the tricuspid valve repair device further includes a third adjusting mechanism configured to adjust an extension length of the push rod with respect to a distal end of the delivery catheter.

The tricuspid valve repair device according to this application can be used for tricuspid valve interventional therapy procedure. When the entire tricuspid valve repair device is delivered to a desired site, the bending angles of the distal end of the guide catheter in the first direction and the second direction are adjusted through the first adjusting mechanism, so that the distal end of the guide catheter approaches the tricuspid valve; the bending angles of the delivery catheter with respect to the guide catheter are adjusted through the second adjusting mechanism, so that the distal end of the delivery catheter further approaches the tricuspid valve. The extension length of the push rod with respect to the distal end of the delivery catheter is adjusted, thereby pushing the push rod to bring the repair clip close to and thus clamp the valve. Thereafter, the repair clip is released, and other parts of the tricuspid valve repair device are withdrawn, thereby finishing the tricuspid valve interventional therapy procedure. The bending adjustments within a large angle in multiple directions in a narrow and complex space are realized, which enlarges the movable space range of the repair clip, expands the repair treatment range of the repair clip on the tricuspid valve, greatly reduces the operation difficulty of the delivery system, and reduces the dependence of the tricuspid valve procedure on the doctor's skill level and clinical experience.

### BRIEF DESCRIPTION OF THE DRAWINGS

In order to more clearly illustrate the technical solutions in the embodiments of the present application, a brief description is given below for the drawings referred in the description of the embodiments. Obviously, the drawings in the following description merely illustrate some embodiments of the application. For those of ordinary skill in the art, other drawings can also be obtained based on the embodiments of the present application and these drawings without involving any inventive effort.
FIG. 1 is a structural schematic view of a tricuspid valve repair device according to a specific embodiment of the present application.
FIG. 2 is a partial structural schematic view of a guide catheter and a first adjusting mechanism in a tricuspid valve repair device according to a specific embodiment of the present application.
FIG. 3 is a partial structural schematic view of a guide catheter and a first adjusting mechanism in a tricuspid valve repair device from a perspective according to a specific embodiment of the present application.
FIG. 4 is a partial structural schematic view of a guide catheter and a first adjusting mechanism in a tricuspid valve repair device from another perspective according to a specific embodiment of the present application.
FIG. 5 is a structural schematic view of a tricuspid valve repair device with a guide catheter and a first adjusting mechanism omitted according to a specific embodiment of the present application.
FIG. 6 is a partial structural schematic view of a delivery catheter and a second adjusting mechanism in a tricuspid valve repair device according to a specific embodiment of the present application.
FIG. 7 is a partial structural schematic view of a delivery catheter and a second adjusting mechanism in a tricuspid valve repair device from a perspective according to a specific embodiment of the present application.
FIG. 8 is a structural schematic view of a guide catheter, a delivery catheter, a push rod, and a repair clip in a first bending state in a tricuspid valve repair device according to a specific embodiment of the present application.
FIG. 9 is a structural schematic view of a guide catheter, a delivery catheter, a push rod, and a repair clip in a second bending state in a tricuspid valve repair device according to a specific embodiment of the present application.
FIG. 10 is a structural schematic view of a guide catheter, a delivery catheter, a push rod, and a repair clip in a third bending state in a tricuspid valve repair device according to a specific embodiment of the present application.

In the drawings:
1- guide catheter; 2- the first adjusting mechanism; 3- delivery catheter; 4- second adjusting mechanism; 5- push rod; 6- third adjusting mechanism; 7- repair clip;
11- first marker band;
21- first operating handle; 22- first adjusting element; 23- first pulling wire; 24- first fixing member; 25- second adjusting member; 26- second pulling wire;
221- first base body; 222- first rotating shaft; 223- first steering part;
31- second marker band;
41- second operating handle; 42- third adjusting member; 43- third pulling wire; 44- second fixing member;
421- third base body; 422- third rotating shaft; 423- third steering part;
61- third operating handle; 62- fourth adjusting member.

### DETAILED DESCRIPTION

In order to make the technical problems solved, technical solutions adopted and technical effects achieved by the present application clearer, the technical solutions of the embodiments of the present application will be further described in detail below with reference to the accompanying drawings. Obviously, the described embodiments are only some but not all of the embodiments of the present application. All other embodiments obtained by those of ordinary skill in the art based on the embodiments of the application without involving creative efforts fall within the protection scope of the application.

In the description of the present application, it is to be understood that orientations or positional relationships indicated by the terms "center", "upper", "lower", "left", "right", "vertical", "horizontal", "inner", "outer", etc. are based on the orientations or positional relationships shown in the drawings. These terms are only intended for facilitating illustrating the present application and simplifying the illustration, rather than indicating or implying that the devices or elements referred thereto have to present a particular orientation, and be constructed and operated in particular orientations, and therefore cannot be construed as limiting the present application. In addition, the terms "first" and "second" are only intended for illustrative purposes, rather than being construed as indicating or implying relative importance. The terms "first position" and "second position" mean two different positions.

In the description of the present application, it is to be noted that unless otherwise expressly specified and defined, the terms "mounted", "coupled" and "connected" should be understood in a broad sense, for example, fixedly connected or detachably connected; or mechanically connected or electrically connected; directly connected or indirectly connected through an intermediate medium, or in an interior communication between two elements. For those of ordinary skill in the art, the specific meanings of the above-described terms in the present application may be understood according to specific circumstances.

In recent years, transcatheter interventional therapy for tricuspid regurgitation has become a research hot spot and a challenge in the field of heart valve disease treatment. Compared with a mitral valve, the tricuspid valve has a larger annulus and valve area, the greatest variability, a more fragile annulus tissue, and more thin leaflets and chordae tendineae. The structures adjacent to the tricuspid valve are complex and prone to damage to the surrounding tissues. The annulus is in semilunar saddle shape, irregular in shape and most prone to dilation, and the degree of tricuspid regurgitation is most likely to be affected by the volume loading. In addition, the right ventricle is in an abundance of myo-trabeculae, columns of muscle and chordae tendineae, and has a weak free wall. The blood flow rate in the right ventricle is low, which prone to thrombus formation, posing a great challenge to the design and application of surgical instruments.

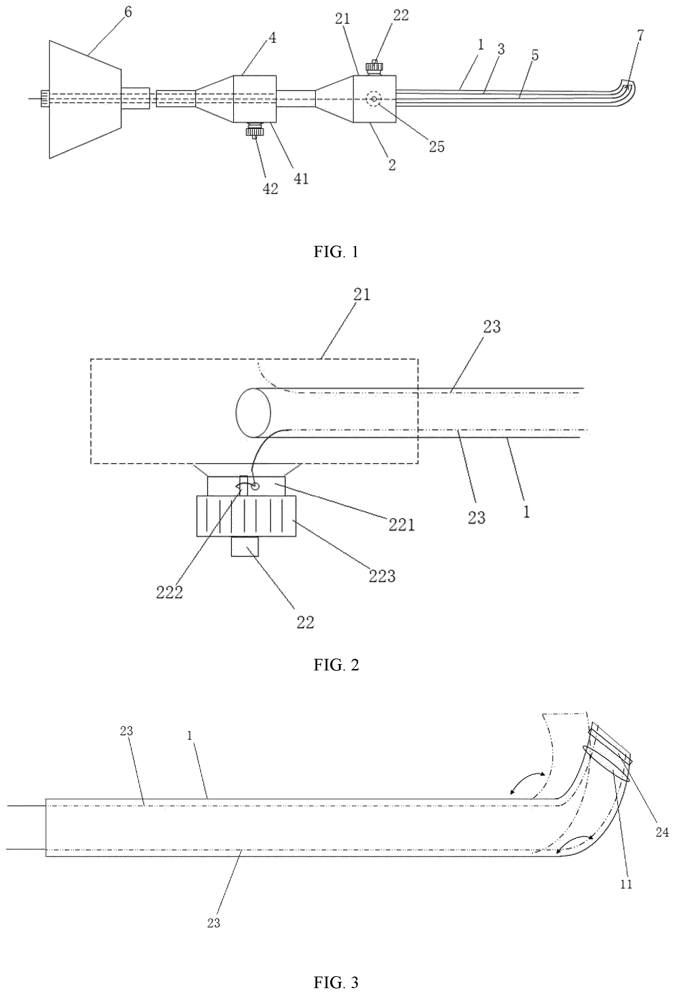

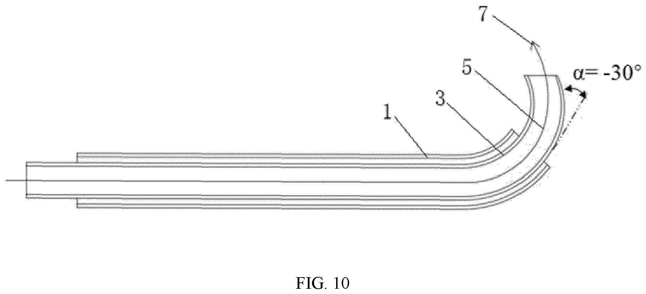

As shown in FIGS. 1 to 10, the present embodiment provides a tricuspid valve repair device, which can be used for tricuspid valve interventional therapy procedure. The tricuspid valve repair device includes a guide catheter 1, a first adjusting mechanism 2, a delivery catheter 3, a second adjusting mechanism 4, a push rod 5, a third adjusting mechanism 6, and a repair clip 7. The first adjusting mechanism 2 is configured to adjust bending angles of a distal end of the guide catheter 1 in a first direction and a second direction. The first direction and the second direction can be perpendicular to each other. The delivery catheter 3 is movably disposed in the guide catheter 1. The second adjusting mechanism 4 is configured to adjust a bending angle of the delivery catheter 3 with respect to the guide catheter 1. The push rod 5 is movably disposed in the delivery catheter 3. The third adjusting mechanism 6 is configured to adjust an extension length of the push rod 5 with respect to a distal end of the delivery catheter 3. The repair clip 7 can be detachably disposed at a distal end of the push rod 5. It can be understood that a proximal end refers to an end closer to the operator (e.g., doctor) and a distal end refers to an end farther away from the operator (e.g., doctor).

A primary pathway for tricuspid valve interventional therapy procedure is usually from inferior vena cava to right atrium, to tricuspid valve, and to right ventricle. When the entire tricuspid valve repair device is delivered to a desired site, the bending angles of the distal end of the guide catheter 1 in the first direction and the second direction are adjusted through the first adjusting mechanism 2, so that the distal end of the guide catheter 1 approaches the tricuspid valve. The bending angle of the delivery catheter 3 with respect to the guide catheter 1 is adjusted through the second adjusting mechanism 4, so that the distal end of the delivery catheter 3 further approaches the tricuspid valve. The extension length of the push rod 5 with respect to the distal end of the delivery catheter 3 is adjusted through the third adjusting mechanism 6, thereby pushing the push rod 5 to bring the repair clip 7 close to and thus clamp the valve. After that, the repair clip 7 is released, and other parts of the tricuspid valve repair device are withdrawn, thereby finishing the tricuspid valve interventional therapy procedure.

Bending adjustment on two planes perpendicular to each other can be realized by the delivery catheter 3, and bending adjustment with respect to the delivery catheter 3 can be realized by the guide catheter 1. The bending adjustment realized by the delivery catheter 3 and the guide catheter 1 enlarges the movable space range of the repair clip 7. For the situation that the position of the posterior and septal valves of the tricuspid valve is lower than the normal position, the delivery catheter 3 and the guide catheter 1 realize the bending adjustments within a large angle in multiple directions in a narrow and complex space, which expands the repair treatment range of the repair clip 7 on the tricuspid valve, greatly reduces the operation difficulty of the delivery system, and reduces the dependence of the tricuspid valve procedure on the doctor's skill level and clinical experience.

Specifically, as shown in FIGS. 1 to 4, the first adjusting mechanism 2 includes a first operating handle 21, a first adjusting member 22, a first pulling wire 23, and a first fixing member 24. A proximal end of the guide catheter 1 is arranged on the first operating handle 21. The first fixing member 24 is disposed at the distal end of the guide catheter 1. At least part of the first pulling wire 23 is disposed inside the guide catheter 1. An end of the first pulling wire 23 is connected to the first adjusting member 22. Another end of the first pulling wire 23 is connected to the first fixing member 24 disposed at the distal end of the guide catheter 1. The first adjusting member 22 is disposed on the first operating handle 21. The first adjusting member 22 is configured to pull or release the first pulling wire 23, and adjust the bending angle of the distal end of the guide catheter 1 in the first direction through the first fixing member 24. During specific operation, one hand holds the first operating handle 21, the other hand rotates the first adjusting member 22, so that the distal end of the guide catheter 1 is pulled by the first pulling wire 23 and the first fixing member 24, to perform a bending action, thereby realizing a bending angle adjustment in the first direction. The operation is simple and quick, time-saving and labor-saving, and the angle and accuracy are controllable, which is beneficial to improve the procedure effect and reduce the procedure requirement. Two first pulling wires 23 may be symmetrically arranged with respect to an axis of the guide catheter 1, and respectively connected to two ends of the first fixing member 24 in a radial direction, which can enhance maneuverability of controlled bend actions. The first fixing member 24 may be made of platinum-tungsten alloy, stainless steel, nickel-titanium alloy or cobalt-chromium alloy. The bending angle of the distal end of the guide catheter 1 in the first direction may be in a range of 0° to 180°, preferably in a range of 0° to 120°.

Further, the first adjusting member 22 can include a first base body 221 and a first rotating shaft 222. The first base body 221 is disposed on the first operating handle 21. The first base body 221 is of a hollow structure. An end of the first rotating shaft 222 is rotatably arranged in the first base body 221. Another end of the first rotating shaft 222 extends out of the first base body 221, and has a first steering part 223 provided thereon. The first pulling wire 23 extends into the first base body 221 and is wrapped on the first rotating shaft 222. During the specific operation, the first steering part 223 is hand-held to rotate the first rotating shaft 222, change the number of wrapping turns of the first pulling wire 23 on the first rotating shaft 222, and thereby change the tension of the first pulling wire 23, so that the distal end of the guide catheter 1 can perform the bending action. The first steering part 223 may be correspondingly provided with a scale, to facilitate checking the angle of rotation during fine adjustment.

In order to enhance a connection strength between the inner wall of the distal end of the guide catheter 1 and the first fixing member 24 and prevent the first fixing member 24 from falling off due to excessive force during controlled bend, optionally, the first fixing member 24 may be a fixing ring. A first limiting protrusion may be formed on one of the inner wall of the distal end of the guide catheter 1 and the first fixing member 24, and a first limiting groove, which matches with the first limiting protrusion, is formed on another one of the inner wall of the distal end of the guide catheter 1 and the first fixing member 24, so as to avoid axial displacement of the first fixing member 24 and the guide catheter 1 relative to each other.

In order to facilitate the observation of the position of the first fixing member 24 inside the human body, optionally, a first marker band 11 abutting the first fixing member 24 is disposed at the distal end of the guide catheter 1, and the first marker band 11 is located on a side of the first fixing member 24 away from the distal end of the guide catheter 1.

The first adjusting mechanism 2 may also include a second adjusting member 25 and a second pulling wire 26. At least part of the second pulling wire 26 is disposed inside the guide catheter 1. An end of the second pulling wire 26 is connected to the second adjusting member 25. Another end of the second pulling wire 26 is connected to the first fixing member 24 disposed at the distal end of the guide catheter 1. The second adjusting member 25 is disposed on the first operating handle 21. The second adjusting member 25 is configured to pull or release the second pulling wire 26, and adjust the bending angle of the distal end of the guide catheter 1 in the second direction through the first fixing member 24. During specific operation, one hand holds the first operating handle 21, the other hand rotates the second adjusting member 25, so that the distal end of the guide catheter 1 is pulled by the second pulling wire 26 and the first fixing member 24, to perform a bending action, thereby realizing a bending angle adjustment in the second direction. The operation is simple and quick, time-saving and labor-saving, and the angle and accuracy are controllable, which is beneficial to improve the procedure effect and reduce the procedure requirement. Two second pulling wires 26 may be symmetrically arranged with respect to an axis of the guide catheter 1, and respectively connected to two ends of the first fixing member 24 in the radial direction, which can enhance maneuverability of controlled bend actions. The bending angle of the distal end of the guide catheter 1 in the second direction may be in a range of 0° to 180°, preferably in a range of 0° to 120°.

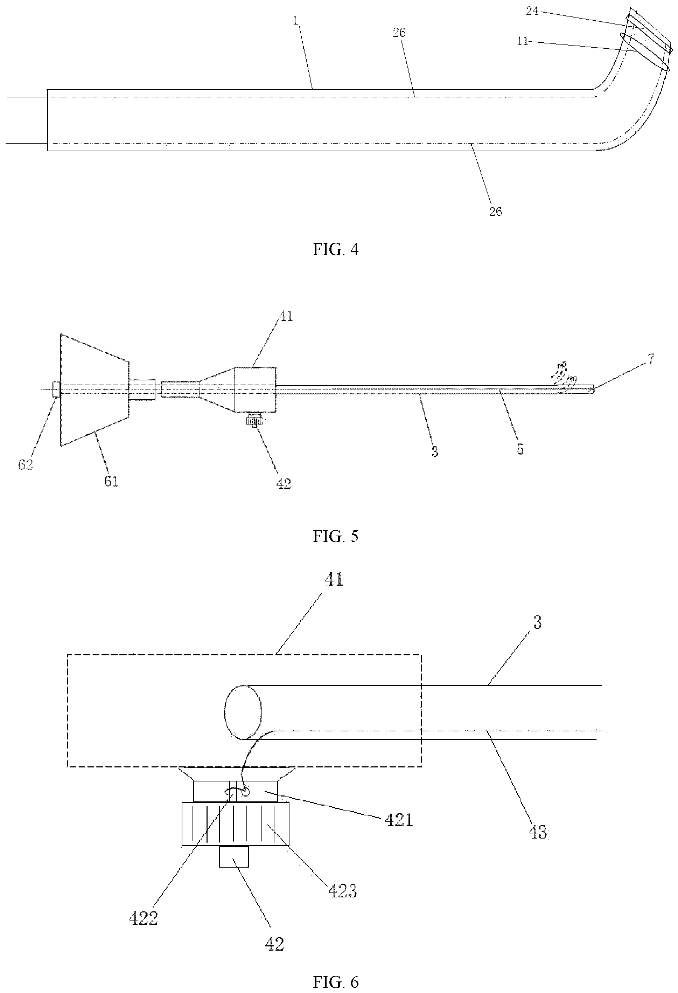

Further, as shown in FIGS. 5 to 7, the second adjusting member 25 can include a second base body and a second rotating shaft. The second base body is disposed on the second operating handle 41. The second base body is of a hollow structure. An end of the second rotating shaft is rotatably arranged in the second base body. Another end of the second rotating shaft extends out of the second base body, and has a second steering part provided thereon. The second pulling wire 26 extends into the second base body and is wrapped on the second rotating shaft. During the specific operation, the second steering part is hand-held, to rotate the second rotating shaft, change the number of wrapping turns of the second pulling wire 26 on the second rotating shaft, and thereby change the tension of the second pulling wire 26, so that the distal end of the guide catheter 1 can perform the bending action. The second steering part may be correspondingly provided with a scale, to facilitate checking the angle of rotation during fine adjustment.

In order to facilitate adjusting the bending angles of the distal end of the guide catheter 1 in the first direction and the second direction, optionally, a plurality of pulling wire cavities extending in an axial direction of the guide catheter 1 are formed on a tube wall of the guide catheter 1. The first pulling wire 23 and the second pulling wire 26 are slidably inserted through the plurality of pulling wire cavities, respectively.

In order to improve the degree of freedom of the distal end of the guide catheter 1, further, the first adjusting mechanism 2 is also configured to adjust the bending angles of the distal end of the guide catheter 1 in a third direction and a fourth direction. The first direction and the third direction are in a first plane. The second direction and the fourth direction are in a second plane. The first plane is perpendicular to the second plane.

Specifically, the first adjusting mechanism 2 may include four pulling wires configured to control the bending angles of the distal end of the guide catheter 1 in the first direction, the second direction, the third direction, and the fourth direction, respectively, so as to meet needs of angle adjustment of the distal end of the guide catheter 1 in different dimensions.

Specifically, the second adjusting mechanism 4 may include a second operating handle 41, a third adjusting member 42, a third pulling wire 43, and a second fixing member 44. A proximal end of the delivery catheter 3 is arranged on the second operating handle 41. The second fixing member 44 is disposed at the distal end of the delivery catheter 3. At least part of the third pulling wire 43 is disposed inside the delivery catheter 3. An end of the third pulling wire 43 is connected to the third adjusting member 42. Another end of the third pulling wire 43 is connected to the second fixing member 44 disposed at the distal end of the delivery catheter 3. The third adjusting member 42 is disposed on the second operating handle 41. The third adjusting member 42 is configured to pull or release the third pulling wire 43, and adjust the bending angle of the delivery catheter 3 with respect to the guide catheter 1 through the second fixing member 44. During specific operation, one hand holds the second operating handle 41, the other hand rotates the third adjusting member 42, so that the distal end of the delivery catheter 3 is pulled by the third pulling wire 43 and the second fixing member 44, to perform a bending action, thereby realizing a bending angle adjustment of the delivery catheter 3 with respect to the guide catheter 1. The procedure requirement is reduced, which is beneficial to improve procedure effect. The operation is simple and quick, time-saving and labor-saving, and the angle and accuracy are controllable. Two third pulling wires 43 may be symmetrically arranged with respect to the axis, and respectively connected to two ends of the second fixing member 44 in the radial direction, which can enhance maneuverability of controlled bend actions. The second fixing member 44 may be made of platinum-tungsten alloy, stainless steel, nickel-titanium alloy or cobalt-chromium alloy. The distal end of the delivery catheter 3 may be bent at an angle in a range of 0° to 180°, preferably in a range of 30° to 150°.

Further, the third adjusting member 42 can include a third base body 421 and a third rotating shaft 422. The third base body 421 is disposed on the third operating handle 61. The third base body 421 is of a hollow structure. An end of the third rotating shaft 422 is rotatably arranged in the third base body 421. Another end of the third rotating shaft 422 extends out of the third base body 421, and has a third steering part 423 provided thereon. The third pulling wire 43 extends into the third base body 421 and is wrapped on the third rotating shaft 422. During the specific operation, the third steering part 423 is hand-held, to rotate the third rotating shaft 422, change the number of wrapping turns of the third pulling wire 43 on the third rotating shaft 422, and thereby change the tension of the third pulling wire 43, thereby realizing a bending angle adjustment of the delivery catheter 3 with respect to the guide catheter 1. The third steering part 423 may be correspondingly provided with a scale, to facilitate checking the angle of rotation during fine adjustment.

In order to enhance a connection strength between the inner wall of the distal end of the delivery catheter 3 and the second fixing member 44 and prevent the second fixing member 44 from falling off due to excessive force during controlled bend, optionally, a second limiting protrusion may be formed on one of the inner wall of the distal end of the delivery catheter 3 and the second fixing member 44, and a second limiting groove, which matches with the second limiting protrusion, is formed on another one of the inner wall of the distal end of the delivery catheter 3 and the second fixing member 44, so as to avoid axial displacement of the second fixing member 44 and the delivery catheter 3 relative to each other.

In order to facilitate the observation of the position of the second fixing member 44 inside the human body, optionally, a second marker band 31 abutting the second fixing member 44 is disposed at the distal end of the delivery catheter 3. The second marker band 31 is located on a side of the second fixing member 44 away from the distal end of the delivery catheter 3.

Specifically, the third adjusting mechanism 6 may include a third operating handle 61 and a fourth adjusting member 62. A proximal end of the push rod 5 is arranged on the third operating handle 61. The fourth adjusting member 62 is disposed on the third operating handle 61, and configured to adjust the extension length of the push rod 5 with respect to the distal end of the delivery catheter 3. The fourth adjusting member 62 and the push rod 5 may form a screw-nut pair. The feeding movement of the push rod 5 is realized by screwing the fourth adjusting member 62.

Optionally, a hardness of a proximal end of the guide catheter 1 is greater than a hardness of a distal end of the guide catheter 1, which greatly reduces the operation difficulty of the delivery system, and reduces the dependence of the tricuspid valve procedure on the doctor's skill level and clinical experience.

Optionally, at a bend portion of the guide catheter 1 in the first direction, a hardness of an inner side of the bend portion of the guide catheter 1 is greater than a hardness of an outer side of the bend portion of the guide catheter 1; and/or at the bend portion in the first direction, a curvature of the guide catheter 1 is greater than a curvature of the delivery catheter 3, so that the bending adjustment of the delivery catheter 3 with respect to the guide catheter 1 during bending adjustment process is not hindered, the bending adjustment difficulty of the delivery catheter 3 is greatly reduced, and the delivery performance is improved.

Optionally, the hardness of the distal end of the guide catheter 1 is equal to or substantially equal to the hardness of the distal end of the delivery catheter 3. When the bending angle of the delivery catheter 3 is relatively small, and the angle between the guide catheter 1 and the delivery catheter 3 is large, the distal end of the delivery catheter 3 is stress-free, thereby avoiding damage to human tissues during delivery.

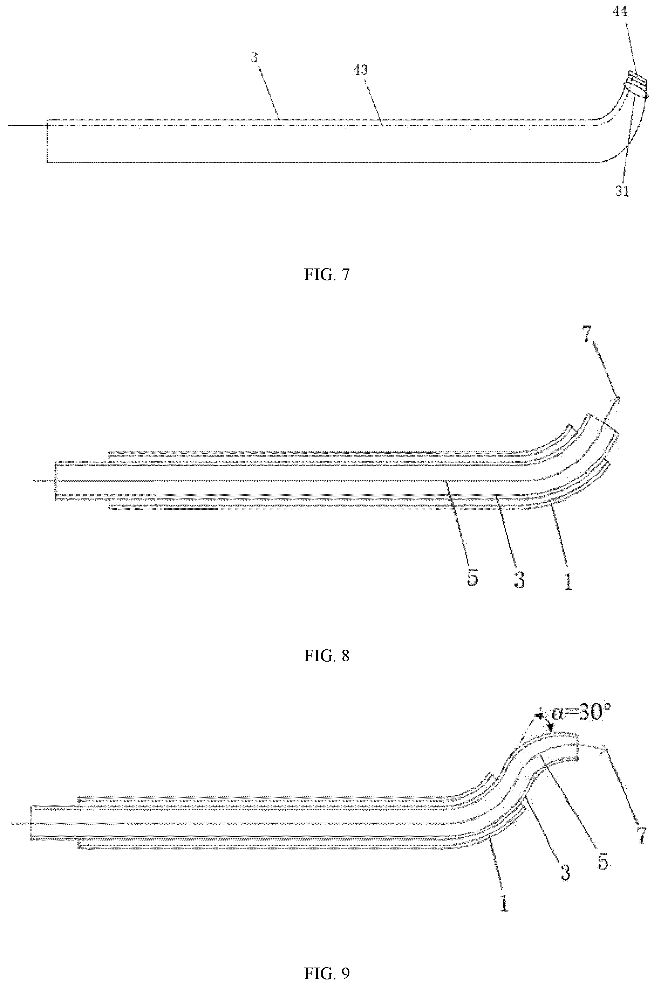

In addition, as shown in FIGS. 8 to 10, the guide catheter 1 and the delivery catheter 3 may or may not be coaxial. When the guide catheter 1 and the delivery catheter 3 are not coaxial, the second adjusting member 25 is adjusted to form an angle between the delivery catheter 3 and the guide catheter 1. The angle between the delivery catheter 3 and the guide catheter 1 is adjustable in a range of -150° to 150°. The angle between the delivery catheter 3 and the guide catheter 1 is adjustable, thereby expanding the range of use of the repair clip 7 to repair the tricuspid valve to a greater extent. For the situation that the position of the posterior and septal valves of the tricuspid valve is lower than the normal position, the delivery catheter 3 and the guide catheter 1 realize a two-stage bending adjustment, which can realize the bending adjustments within a large angle in multiple directions in a narrow and complex space, enlarges the movable space range of the repair clip 7, and expands the repair treatment range of the repair clip 7 on the tricuspid valve.

The tricuspid valve repair device according to this embodiment can be used for tricuspid valve interventional therapy procedure, and the procedure is generally as follows. A primary pathway for tricuspid valve interventional therapy procedure is usually from inferior vena cava to right atrium, to tricuspid valve, and to right ventricle. When the whole tricuspid valve repair device is delivered to a desired site, the first operating handle 21 is hand-held, to rotate the first adjusting member 22, so that the distal end of the guide catheter 1 is pulled by the first pulling wire 23 and the first fixing member 24 to perform the bending action, thereby realizing the bending angle adjustment in the first direction. Then, the first operating handle 21 is hand-held, to rotate the second adjusting member 25, so that the distal end of the guide catheter 1 is pulled by the second pulling wire 26 and the first fixing member 24 to perform the bending action, thereby realizing the bending angle adjustment in the second direction, so that the distal end of the guide catheter 1 approaches the tricuspid valve. Then, the second operating handle 41 is hand-held, to rotate the third adjusting member 42, so that the distal end of the delivery catheter 3 is pulled by the third pulling wire 43 and the second fixing member 44 to perform the bending action, thereby realizing the bending angle adjustment of the delivery catheter 3 with respect to the guide catheter 1, so that the distal end of the delivery catheter 3 further approaches the tricuspid valve. Then, the fourth adjusting member 62 is rotated, to realize the feeding movement of the push rod 5. As such, the push rod 5 is pushed to bring the repair clip 7 close to and thus clamp the valve. Thereafter, the repair clip 7 is released, and other parts of the tricuspid valve repair device are withdrawn, thereby finishing the tricuspid valve interventional therapy procedure.

It should be noted that, only the preferred embodiments and technical principles used in this application are illustrated in the above. Those skilled in the art will understand that the present application is not limited to the specific embodiments herein, and those skilled in the art can make various obvious changes, readjustments and substitutions without departing from the protection scope of the present application. Therefore, although the above embodiments have provided a more detailed illustration of the present application, the present application is not limited to the above embodiments. Without departing from the concept of the present application, the present application may also include more other equivalent embodiments, and the scope of the present application shall be subjected to the appended claims.

## Claims

1. A tricuspid valve repair device, comprising:
a guide catheter (1);
a first adjusting mechanism (2) configured to adjust bending angles of a distal end of the guide catheter (1) in a first direction and a second direction;
a delivery catheter (3) movably disposed in the guide catheter (1);
a second adjusting mechanism (4) configured to adjust a bending angle of the delivery catheter (3) with respect to the guide catheter (1);
a push rod (5) movably disposed in the delivery catheter (3); and
a repair clip (7) detachably disposed at a distal end of the push rod (5).

2. The tricuspid valve repair device according to claim 1, wherein the first adjusting mechanism (2) comprises a first operating handle (21), a first adjusting member (22) and a first pulling wire (23); a proximal end of the guide catheter (1) is arranged on the first operating handle (21); an end of the first pulling wire (23) is connected to the first adjusting member (22), another end of the first pulling wire (23) is connected to the distal end of the guide catheter (1); the first adjusting member (22) is arranged on the first operating handle (21); and the first adjusting member (22) is configured to pull or release the first pulling wire (23) to adjust the bending angle of the distal end of the guide catheter (1) in the first direction.

3. The tricuspid valve repair device according to claim 2, wherein the first adjusting mechanism (2) further comprises a first fixing member (24) disposed at the distal end of the guide catheter (1), and the other end of the first pulling wire (23) is connected to the first fixing member (24).

4. The tricuspid valve repair device according to claim 3, wherein two first pulling wires (23) are symmetrically arranged with respect to an axis of the guide catheter (1), and are respectively connected to two ends of the first fixing member (24) in a radial direction.

5. The tricuspid valve repair device according to claim 3, wherein the first fixing member (24) is a fixing ring; a first limiting protrusion is formed on one of an inner wall of the distal end of the guide catheter (1) and the first fixing member (24), and a first limiting groove, which matches with the first limiting protrusion, is formed on another one of the inner wall of the distal end of the guide catheter (1) and the first fixing member (24).

6. The tricuspid valve repair device according to claim 3, wherein a first marker band (11) abutting the first fixing member (24) is disposed at the distal end of the guide catheter (1); the first marker band (11) is located on a side of the first fixing member (24) away from the distal end of the guide catheter (1).

7. The tricuspid valve repair device according to claim 2, wherein the first adjusting member (22) comprises a first base body (221) and a first rotating shaft (222); the first base body (221) is disposed on the first operating handle (21); the first base body (221) is of a hollow structure; an end of the first rotating shaft (222) is rotatably arranged in the first base body (221), another end of the first rotating shaft (222) extends out of the first base body (221), and has a first steering part (223) provided thereon; the first pulling wire (23) extends into the first base body (221) and is wrapped on the first rotating shaft (222).

8. The tricuspid valve repair device according to claim 7, wherein the first steering part (223) is correspondingly provided with a scale.

9. The tricuspid valve repair device according to claim 2, wherein the first adjusting mechanism (2) further comprises a second adjusting member (25) and a second pulling wire (26), and wherein an end of the second pulling wire (26) is connected to the second adjusting member (25), another end of the second pulling wire (26) is connected to the distal end of the guide catheter (1); the second adjusting member (25) is arranged on the first operating handle (21); and the second adjusting member (25) is configured to pull or release the second pulling wire (26) to adjust the bending angle of the distal end of the guide catheter (1) in the second direction.

10. The tricuspid valve repair device according to claim 9, wherein a plurality of pulling wire cavities extending in an axial direction of the guide catheter (1) are formed on a tube wall of the guide catheter (1); and the first pulling wire (23) and the second pulling wire (26) are slidably inserted through the plurality of pulling wire cavities, respectively.

11. The tricuspid valve repair device according to claim 9, wherein the second adjusting member (25) comprises a second base body and a second rotating shaft, and wherein the second base body is disposed on the second operating handle (41); the second base body is of a hollow structure; an end of the second rotating shaft is rotatably arranged in the second base body, another end of the second rotating shaft extends out of the second base body, and has a second steering part provided thereon; the second pulling wire (26) extends into the second base body and is wrapped on the second rotating shaft.

12. The tricuspid valve repair device according to claim 9, wherein the first adjusting mechanism (2) further comprises a first fixing member (24) disposed at the distal end of the guide catheter (1); and two second pulling wires (26) are symmetrically arranged with respect to an axis of the guide catheter (1), and are respectively connected to two ends of the first fixing member (24) in a radial direction.

13. The tricuspid valve repair device according to claim 1, wherein a hardness of a proximal end of the guide catheter (1) is greater than a hardness of the distal end of the guide catheter (1).

14. The tricuspid valve repair device according to claim 1, wherein at a bend portion in the first direction, a hardness of an inner side of the bend portion of the guide catheter (1) is greater than a hardness of an outer side of the bend portion of the guide catheter (1).

15. The tricuspid valve repair device according to claim 1, wherein at a bend portion in the first direction, a curvature of the guide catheter (1) is greater than a curvature of the delivery catheter (3).

16. The tricuspid valve repair device according to claim 1, wherein the first adjusting mechanism (2) is further configured to adjust bending angles of the distal end of the guide catheter (1) in a third direction and a fourth direction; the first direction and the third direction are in a first plane, the second direction and the fourth direction are in a second plane, and the first plane is perpendicular to the second plane.

17. The tricuspid valve repair device according to claim 16, wherein the first adjusting mechanism (2) comprises four pulling wires configured for controlling the bending angles of the distal end of the guide catheter (1) in the first direction, the second direction, the third direction, and the fourth direction, respectively.

18. The tricuspid valve repair device according to any one of claims 1 to 17, wherein the second adjusting mechanism (4) comprises a second operating handle (41), a third adjusting member (42) and a third pulling wire (43); a proximal end of the delivery catheter (3) is arranged on the second operating handle (41); an end of the third pulling wire (43) is connected to the third adjusting member (42), another end of the third pulling wire (43) is connected to a distal end of the delivery catheter (3); the third adjusting member (42) is arranged on the second operating handle (41); and the third adjusting member (42) is configured to pull or release the third pulling wire (43) to adjust a bending angle of the delivery catheter (3) with respect to the guide catheter (1).

19. The tricuspid valve repair device according to claim 18, wherein the third adjusting member (42) comprises a third base body (421) and a third rotating shaft (422), and wherein the third base body (421) is disposed on the third operating handle (61); the third base body (421) is of a hollow structure; an end of the third rotating shaft (422) is rotatably arranged in the third base body (421), another end of the third rotating shaft (422) extends out of the third base body (421), and has a third steering part (423) provided thereon; the third pulling wire (43) extends into the third base body (421) and is wrapped on the third rotating shaft (422).

20. The tricuspid valve repair device according to any one of claims 1 to 17, wherein the tricuspid valve repair device further comprises a third adjusting mechanism (6) configured to adjust an extension length of the push rod (5) with respect to a distal end of the delivery catheter (3).
